# EUROPEAN PATENT APPLICATION

(11) **EP 4 567 384 A1**
(43) Date of publication of application: **11.06.2025**
(21) Application number: 24214209.9
(22) Date of filing: 20.11.2024
(51) Int. Cl.: G01F 22/00, G01F 23/30, G01F 23/00, B65B 55/04

(54) **ARRANGEMENT FOR MEASURING A VOLUME OF STERILIZING SOLUTION USED PER TIME UNIT AND A METHOD THEREOF**

(30) Priority: 06.12.2023 EP 23214736
(71) Applicant: Tetra Laval Holdings & Finance S.A., 1009 Pully (CH)
(72) Inventor: Svenle, Martin, 22186 Lund (SE); Karlsson, Björn, 22186 Lund (SE); Saeidihaghi, Arash, 22186 Lund (SE)
(74) Representative: Tetra Pak - Patent Attorneys SE

(57) **Abstract**

An arrangement (100) for measuring a volume (VOL) of sterilizing solution (102) used per time unit in a roll-based packaging machine (104), said arrangement (100) comprising a measuring tank (106) fluidly connected to a sterilizing solution bath (108), a measuring tank level detection device (110) arranged to generate measuring tank level data (112) representing a measuring tank level (MT-L), a level tank (114) fluidly connected to the sterilizing solution bath (108) in at least two points (116, 118) such that the level tank (114) and the sterilizing solution bath (108) constitute communicating vessels, a level tank level detection device (120) arranged to generate level tank level data (122) representing a level tank level (LT-L), a measuring tank valve (124) placed on a lower measuring tank pipe (126) connecting the measuring tank (106) to the bath (108), wherein in an open state (OS) the sterilizing solution in the measuring tank (106) is allowed to pass into the bath (108), and in a closed state (CS) the sterilizing solution in the measuring tank (106) is hindered to pass, and a control unit (128) configured to retrieve the level tank level data (122), in case the level tank level (LT-L) is equal to or below a lower reference level (LOW-REF), to open the measuring tank valve (124) such that at least part of the sterilizing solution (102) in the measuring tank (106) is transferred to the bath (108), and to register a time point for opening the measuring tank valve (124) as a filling start time point, in case the level tank level (LT-L) is equal to or above an upper reference level (UPP-REF), to close the measuring tank valve (124) such that the sterilizing solution (102) from the measuring tank (106) is hindered from being transferred to the bath (108), and to register a time point for closing the measuring tank valve (124) as a filling end time point to determine a filling start measuring tank level by retrieving the measuring the level tank level (LT-L) for the filling start time point, to determine a filling end measuring tank level (MT-L) by retrieving the measuring tank level data (112) for the filling end time point, to determine the volume of sterilizing solution used between the filling start time point and the filling end time point by using the measuring tank level data (112) for the filling start time point and the filling end time point, and a cross-sectional area of the measuring tank (106), and to determine the volume of sterilizing solution used per time unit by dividing the volume of sterilizing solution determined with a number of time units between the filling start time point and the filing end time point.

## Description

### Technical Field

The invention relates to packaging technology. More particularly, it is related to arrangements and methods for sterilization of a web of packaging material.

### Background Art

Since many years, it is well known to use so-called hydrogen peroxide baths in roll-fed filling machines, sometimes referred to as packaging machines. During operation, a web of packaging material is fed down into the bath such that bacteria, germs and other unwanted microorganisms are killed off. As an effect of this, the risk of having food product being contaminated at a later stage when the packaging material is formed into a package and the food product is filled into the package is reduced.

After the web has been in the bath, excessive hydrogen peroxide is removed from the web by having this fed through a dryer or similar device. By having the excessive hydrogen peroxide removed, the web is prepared and ready for filling and sealing. Further, by drying the web, it can also be assured that there is no hydrogen peroxide left on the web before this is formed into the package and filled with the food product.

Different solutions have been developed over the years for assuring that there is enough hydrogen peroxide used to assure sterilization, but not that too much hydrogen peroxide is being used such that there is a risk that there is hydrogen peroxide is not removed during drying such that this ends up in the food product.

One way of solving the problem, in other words striking the balance of neither underusing and overusing hydrogen peroxide, is to increase tolerances. For instance, by increasing a web path in the hydrogen peroxide the risk of not using enough hydrogen peroxide to kill off microorganisms can be reduced. In a similar manner, a web path in the dryer may be extended to assure the excessive hydrogen peroxide is removed adequately.

Since increasing the tolerances comes with a cost, e.g. that a total web path in the filling machine is extended and as an effect that losses increases during a machine stop, other solutions to the problem have been developed. One such solution is to have the hydrogen peroxide bath placed on a weighing device. By being able to monitor the weight of the bath continuously, it is made possible to determine a consumption of hydrogen peroxide as a function of time.

Using the weighing device as described above has the effect that information about the consumption is provided. However, even though there is a solution to the problem, there is a need for a solution for being able to in an improved manner track the consumption of hydrogen peroxide or any other sterilizing solution used during operation of the filling machine.

### Summary

It is an object of the invention to at least partly overcome one or more of the above-identified limitations of the prior art. In particular, it is an object to provide a more precise as well as more reliable measurement of the consumption of sterilizing solution, such as hydrogen peroxide. By doing so, it is made possible to further improve the packaging machines such that sufficient hydrogen peroxide, or other sterilizing solution, is used for assuring packaging material sterility but not too much such that there is a risk that there is hydrogen peroxide residues in the packages produced.

According to a first aspect it is provided an arrangement for measuring a volume of sterilizing solution used per time unit in a roll-based packaging machine, said arrangement may comprise
a measuring tank fluidly connected to a sterilizing solution bath,
a measuring tank level detection device arranged to generate measuring tank level data representing a measuring tank level,
a level tank fluidly connected to the sterilizing solution bath in at least two points such that the level tank and the sterilizing solution bath constitute communicating vessels,
a level tank level detection device arranged to generate level tank level data representing a level tank level,
a measuring tank valve placed on a lower measuring tank pipe connecting the measuring tank to the bath, wherein in an open state the sterilizing solution in the measuring tank is allowed to pass into the bath, and in a closed state the sterilizing solution in the measuring tank is hindered to pass, and
a control unit configured
to retrieve the level tank level data,
in case the level tank level is equal to or below a lower reference level,
   to open the measuring tank valve such that at least part of the sterilizing solution in the measuring tank is transferred to the bath, and
   to register a time point for opening the measuring tank valve as a filling start time point,
in case the level tank level is equal to or above an upper reference level,
   to close the measuring tank valve such that the sterilizing solution from the measuring tank is hindered from being transferred to the bath, and
   to register a time point for closing the measuring tank valve as a filling end time point,
to determine a filling start measuring tank level by retrieving the measuring the level tank level for the filling start time point,
to determine a filling end measuring tank level by retrieving the measuring tank level data for the filling end time point,
to determine the volume of sterilizing solution used between the filling start time point and the filling end time point by using the measuring tank level data for the filling start time point and the filling end time point, and a cross-sectional area of the measuring tank, and
to determine the volume of sterilizing solution used per time unit by dividing the volume of sterilizing solution determined with a number of time units between the filling start time point and the filing end time point.

There are several reasons why the concept suggested herein may be preferred over the weighing device used today. One reason is that the weighing device is not able to respond in a timely manner. In roll-fed filling machines it is namely common that the web moves at a speed of 2.5 to 7 meter per second (150 to 420 meter per minute). In addition, this speed may also vary over time. The effect of having the web transferred at this speed, and also by having the speed varying over time, is that using the weighing device for estimating the consumption of sterilizing solution provides insufficient accuracy.

By having the level tank connected to the bath as described above, the level in the bath may be measured indirectly. Put differently, the negative effects caused by the moving web on the measurements may be overcome.

The measuring tank may be placed above the sterilizing solution bath such that with the measuring tank valve in the open state the sterilizing solution in the measuring tank is released down into the sterilizing solution bath via the valve, and wherein the measuring tank valve may be configured such that in case there is no electrical power provided, the valve is in the open state.

By having the valve configured as described above, in case of an electric power failure, sterilizing solution will be provided down into the bath to assure that this does not run dry.

The measuring tank level detection device and/or the level tank level detection device may be a floating device.

The sterilizing solution may be hydrogen peroxide.

A total volume of the bath may be 5-20 times a total volume of the level tank.

A total volume of the measuring tank may be greater than the total volume of the level tank.

The volume of sterilizing solution used per time unit may be 0.3 to 2 liters per hour.

The control unit may be further configured to
in case the volume of sterilizing solution per time unit is below a lower risk threshold,
to transmit a sterility risk notification to an operator device.

An advantage with this is an operator can get a notification that the consumption of sterilizing solution does not meet a set range. Put differently, by receiving such notification, there may be a reason for the operator to inspect the packaging machine and/or the arrangement to assure that it is operating adequately.

The control unit may be further configured to
in case the volume of sterilizing solution per time unit is above an upper risk threshold,
to transmit a sterilizing solution residues risk notification to the operator device.

Similar to the notification described above, by notifying that the consumption is not within the set range, the operator may get an early indication that there is a risk that packages not fulfilling set quality standards are being produced. For instance, the sterilizing residues risk notification may suggest to look into the produced packages to assure that these do not contain sterilizing solution residues.

According to a second aspect it is provided a roll-fed packaging machine comprising the arrangement according to the first aspect.

According to a third aspect it is provided a method for measuring a volume of sterilizing solution used per time unit in a roll-based packaging machine by using an arrangement, said arrangement may comprise a measuring tank fluidly connected to a sterilizing solution bath, a measuring tank level detection device arranged to generate measuring tank level data representing a measuring tank level, a level tank fluidly connected to the sterilizing solution bath in at least two points such that the level tank and the sterilizing solution bath constitute communicating vessels, a level tank level detection device arranged to generate level tank level data representing a level tank level, a measuring tank valve placed on a lower measuring tank pipe connecting the measuring tank to the bath, wherein in an open state the sterilizing solution in the measuring tank is allowed to pass into the bath, and in a closed state the sterilizing solution in the measuring tank is hindered to pass, and a control unit, said method may comprise
retrieving the level tank level data,
in case the level tank level is equal to or below a lower reference level,
   opening the measuring tank valve such that at least part of the sterilizing solution in the measuring tank is transferred to the bath, and
   registering a time point for opening the measuring tank valve as a filling start time point,
in case the level tank level is equal to or above an upper reference level,
   closing the measuring tank valve such that the sterilizing solution from the measuring tank is hindered from being transferred to the bath, and
   registering a time point for closing the measuring tank valve as a filling end time point,
determining a filling start measuring tank level by retrieving the measuring level tank level for the filling start time point,
determining a filling end measuring tank level by retrieving the measuring tank level data for the filling end time point,
determining the volume of sterilizing solution used between the filling start time point and the filling end time point by using the measuring tank level data for the filling start time point and the filling end time point, and a cross-sectional area of the measuring tank, and
determining the volume of sterilizing solution used per time unit by dividing the volume of sterilizing solution determined with a number of time units between the filling start time point and the filing end time point.

The same advantages and features as presented with respect to the first aspect also apply to this aspect.

A total volume of the bath may be 5-20 times a total volume of the level tank.

The method may further comprise
in case the volume of sterilizing solution per time unit is below a lower risk threshold,
transmitting a sterility risk notification to an operator device.

The method may further comprise
in case the volume of sterilizing solution per time unit is above an upper risk threshold,
transmitting a sterilizing solution residues risk notification to the operator device.

According to a fourth aspect it is provided a computer program product comprising instructions which, when the program is executed by a computing device, causes the computer to carry out the method according to the third aspect.

Still other objectives, features, aspects and advantages of the invention will appear from the following detailed description as well as from the drawings.

### Brief Description of the Drawings

Embodiments of the invention will now be described, by way of example, with reference to the accompanying schematic drawings, in which
Fig. 1A is a general illustration of an arrangement for measuring a volume of sterilizing solution used per time unit.
Fig. 1B is a cross-sectional view of a level tank forming part of the arrangement illustrated in fig. 1A.
Fig. 1C is a cross-sectional view of a measuring tank forming part of the arrangement illustrated in fig. 1A.
Fig. 2 is a flowchart illustrating a method for measuring a volume of sterilizing solution used per time unit.
Fig. 3 is a diagram illustrating a level tank level over time.

### Detailed Description

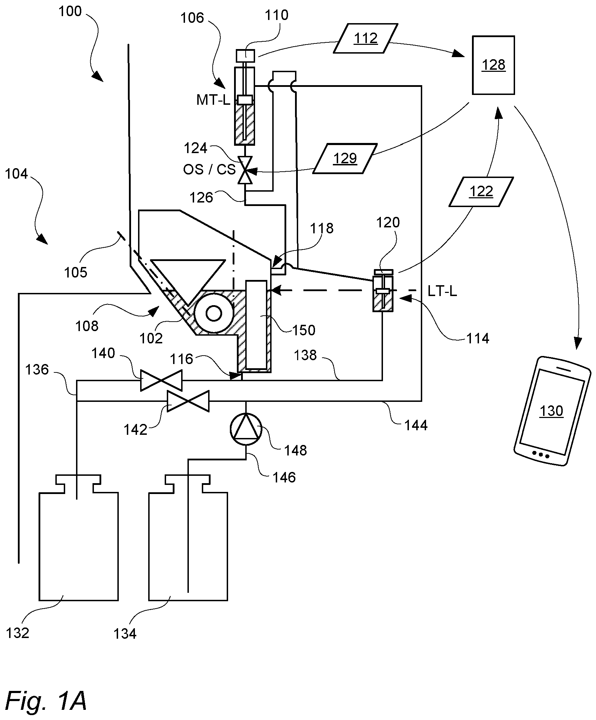

With reference to Fig. 1A an arrangement 100 for measuring a volume of sterilizing solution 102 used per time unit is illustrated. The arrangement 100 can form part of a roll-fed packaging machine 104, that is, a packaging machine in which a web 105 of packaging material is transformed into packages filled with food product or the like. An example of such machine is Tetra Pak^{™} A3 producing Tetra Brik^{™} packages marketed by Tetra Pak^{™}.

As illustrated, the web 105 can be fed into a sterilizing solution bath 108. A roller may be provided in the bath to assure that the web is guided adequately. Different sterilizing solutions may be used. By way of example, the sterilizing solution may be hydrogen peroxide.

The reason for having the web 105 fed into the bath 108 is to assure that unwanted microorganisms are killed off. To make sure that excessive sterilizing solution is removed from the web downstream the bath, a drying device may be provided (not illustrated). The drying device may comprise a number of heating elements to provide for that the excessive sterilizing solution is removed. Alternatively, or in addition, a web path downstream the bath and upstream a tube forming arrangement, that is, an arrangement arranged to form a tube of the web by attaching longitudinal edges of the web to each other, may be arranged such that the excessive sterilizing solution can be removed by air drying, i.e. no active drying of the web.

As illustrated, a measuring tank 106 may be placed above the bath 108. The measuring tank 106 may be provided with a measuring tank level detection device 110, e.g. a floating device as illustrated. By having this device, it is made possible to trace a difference in measuring tank level MT-L over time. As an effect of this, by combining this with a cross-sectional area CS-A of the measuring tank 106, a volume VOL provided into the bath 108 from the measuring tank 106 from a first time point 1^{st} TP to a second time point 2^{nd} TP can be determined.

Information about a measuring tank level MT-L measured by the measuring tank level detection device 110 may be provided to a control unit 128 via measuring tank level data 112. This information may be provided both via wire and/or via wireless transmission. The control unit 128 may form part of the packaging machine 104 or it may be placed remotely, i.e. external to the packaging machine 104.

A level tank 114 can be provided next to the bath 108. This can be fluidly connected to the bath 108 in a first and a second point 116, 118, respectively. The first point 116 may be in a bottom part of the bath 108 and the second point 118 may be in a top part of the bath 108, as illustrated. By having the bath 108 and the level tank 114 connected in this way, the level tank 114 and the bath 108 will constitute communicating vessels. As an effect of this, a level of the sterilizing solution 102 in the bath 108 is reflected in a level tank level LT-L in the level tank 114.

The level tank 114 may be equipped with a level tank level detection device 120, e.g. a floating device. Level tank level data 122, generated by the level tank level detection device 120, may be transferred to the control unit 128, wherein the level tank level data 122 pertains to the level tank level LT-L.

Once the measuring tank level data 112 and the level tank level data 122 have been processed by the control unit 128, control data 129 can be transferred to a measuring tank valve 124 placed on a lower measuring tank pipe 126. The lower measuring tank pipe 126 may connect the measuring tank 106 with the bath 108.

More particularly, the processing of the level tank level data 122 and the measuring tank level data 112 may comprise; in case the level tank level LT-L is equal to or below a lower reference level LOW-REF, to open the measuring tank valve 124 such that at least part of the sterilizing solution 102 in the measuring tank 106 is transferred to the bath 108, and to register a time point for opening the measuring tank valve 124 as a filling start time point, i.e. first time point 1^{st} TP. On the other hand, in case the level tank level LT-L is equal to or above an upper reference level UPP-REF, to close the measuring tank valve 124 such that the sterilizing solution 102 from the measuring tank 106 is hindered from being transferred to the bath 108, and to register a time point for closing the measuring tank valve 124 as a filling end time point, i.e. a second time point 2^{nd} TP. A filling start measuring tank level can be determined by retrieving the measuring the level tank level LT-L for the filling start time point. Similarly, a filling end measuring tank level MT-L can be determined by retrieving the measuring tank level data 112 for the filling end time point. The volume of sterilizing solution used between the filling start time point and the filling end time point can be determined by using the measuring tank level data 112 for the filling start time point and the filling end time point, and the cross-sectional area CS-A of the measuring tank 106. The volume of sterilizing solution used per time unit can be determined by dividing the volume of sterilizing solution with a number of time units between the filling start time point and the filing end time point.

An advantage of using the level tank 120 for measuring the level tank level LT-L, instead of measuring this directly in the bath 108, is that the effects of feeding the web 105 at a speed of 2.5 to 7 meter per second, or more, in the bath 108 can be reduced. Put differently, reliable level measurements are difficult to achieve when having waves formed by the moving web. This is however solved by using the level tank 114 connected to the bath 108 as described above.

The measuring tank valve 124 may be configured such that, in case there is no electrical power provided to the valve, this is in an open state OS, thereby providing for that the sterilizing solution in the measuring tank 106 is released down into the sterilizing solution bath 108 via the valve 124. A benefit of this is that in case there is an electrical power failure, this will not result in that the bath 108 will run dry, thereby risking that the web 105 is not sufficiently sterilized.

A total volume of the bath 108 may be 5-20 times a total volume of the level tank 114. Since the level in the bath 108 will only deviate within a relatively small range, compared to the total volume of the bath, the level tank 114 can be made to hold a lower volume than the bath. Further, a total volume of the measuring tank 106 may be greater than the total volume of the level tank.

As illustrated, the control unit 128 can be configured to, in case the volume of sterilizing solution per time unit is below a lower risk threshold LOW-RISK, to transmit a sterility risk notification to an operator device 130, herein exemplified by a mobile phone. Put differently, in case the volume of sterilizing solution is so low that there is a risk that the web 105 is not sufficiently exposed to the sterilizing solution, this may be detected, and also made the operator aware of. The lower risk threshold LOW-RISK may be dynamically adjusted to the web speed.

In a similar manner, the control unit 128 can be configured to, in case the volume of sterilizing solution per time unit is above an upper risk threshold UPP-RISK, to transmit a sterilizing solution residues risk notification to the operator device 130. In other words, in this way it can be detected if there is, for whatever reason, too much sterilizing solution in the bath such that there is a risk that the excessive sterilizing solution is not adequately removed. The upper risk threshold UPP-RISK may be dynamically adjusted to the web speed.

As illustrated, a used sterilizing solution tank 132 and a new sterilizing solution 134 may be provided. A bath pipe 136 can be used for connecting the bath 108 to the used sterilizing solution tank 132, and a level tank pipe 138 can be used for connecting the level tank 114 to the used sterilizing solution tank 132. As illustrated, the level tank pipe 138 may feed into the bath pipe 136. Downstream from where the two coincide, a first used sterilizing solution valve 140 may be placed. When having this valve opened, sterilizing solution is released from the bath and the level tank down into the used sterilizing solution tank 132. A second used sterilization solution valve 142 may be placed on an upper measuring tank pipe 144. This pipe provides for that the sterilizing solution in the measuring tank 106 can be released down into the used sterilizing solution tank 132 when the second used sterilizing solution is opened.

New sterilizing solution may be fed from the new sterilizing solution tank 134 to the measuring tank 106 via the new sterilizing solution pipe 146 and the upper measuring tank pipe 144. A new sterilizing solution pump 148 can be provided on the new sterilizing solution pipe 146. When filling new sterilizing solution into the measuring tank 106, the first and second used sterilizing solution valves 140, 142 can be closed. When releasing used sterilizing solution from the measuring tank 106 into the used sterilizing solution tank 132, the pump 148 can be deactivated, and hindering the used sterilizing solution from reaching the new sterilizing solution tank 134.

Further, a level adjusting body 150 may be provided. During production, the level adjusting body 150 may be placed down in the bath 108, as illustrated. During a stop, to prevent that the web 105 is standing still in the sterilizing solution 102, the level adjusting body 150 may be lifted up from the bath 108 resulting in that a level of the sterilizing solution 102 is lowered below a lowermost part of the web. In this way, negative effects of having the web standing still in the sterilizing solution can be prevented. This in turn has the positive effect that there is no need to have sections of the web discarded after the stop, which most often is the case when there is no possibility of lowering the level of the sterilizing solution during the stop. Having the possibility of lowering the level during the stop by using the level adjusting body 150 in combination with using the level tank 114 fluidly connected to the bath 108 comes with the positive effect that the level in the bath can be reliably measured even though the bath 108 may house the level adjusting body 150 and/or other elements. In other words, it is an advantage of using the level tank 114 since there may not be space available for measuring the level of the sterilizing solution directly in the bath 108. One reason for the lack of space may be that the level adjusting body 150 is provided in or above the bath 108.

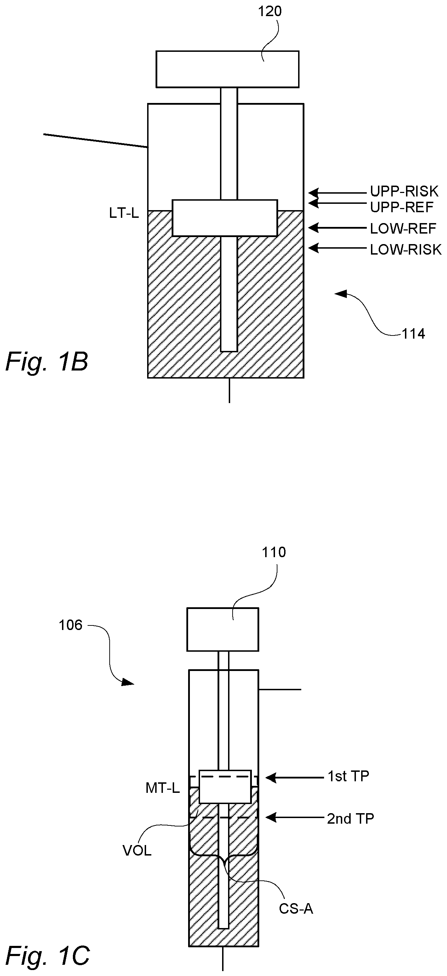

Fig. 1B illustrates the level tank 114 provided with the level tank level detection device 120 in further detail. As described above, when the level tank level LT-L reaches the lower reference level LOW-REF, sterilizing solution may be added into the bath 108 and indirectly into the level tank 114. On the other hand, when the level tank level LT-L reaches the upper reference level UPP-REF, filling sterilizing solution into the bath 108 can be ended. In case the lower risk threshold level LOW-RISK is reached, the sterility risk notification may be issued. Further, in case the upper risk threshold level UPP-RISK is reached, the sterilizing solution residues risk notification may be issued.

Instead of having the filling start time point being triggered by that the lower reference level LOW-REF is reached, the filling start time point may be a set time point. In the same manner, the filling end time point may also be a set time point instead of being triggered by that the upper reference level UPP-REF is reached. Even if having these two time points being set, i.e. the time difference between the time points are set, the consumption can be determined due to that the level in the level tank is determined at the two different time points.

Fig. 1C illustrates the measuring tank 106 in further detail. As described above, the volume VOL released from the measuring tank 106 can be determined by taking into account the level in the tank at the first time point 1^{st} TP, i.e. the filling start time point, the level in the tank at the second time point 2^{nd} TP, i.e. the filling end time point, and the cross-sectional area CS-A. The filling start time point may occur when the level tank level LT-L reaches the lower reference level LOW-REF, and the filling end time point may occur when the level tank level LT-L reaches the upper reference level UPP-REF.

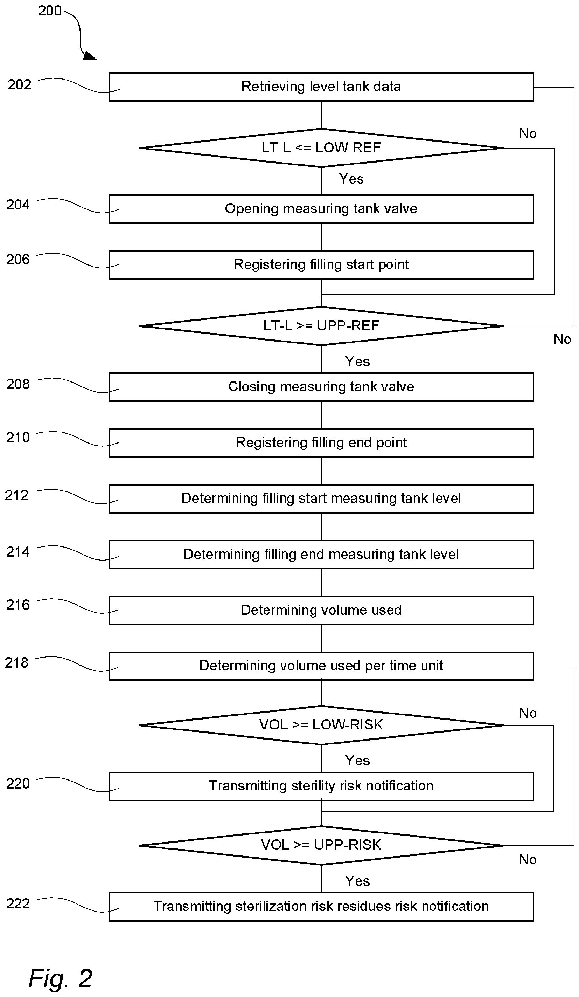

Fig. 2 is a flowchart illustrating a method 200 for measuring the volume VOL of the sterilizing solution 102 used per time unit in the roll-based packaging machine 104. The method can comprise
retrieving 202 the level tank level data 122,
in case the level tank level LT-L is equal to or below the lower reference level LOW-REF,
   opening 204 the measuring tank valve 124 such that at least part of the sterilizing solution 102 in the measuring tank 106 is transferred to the bath 108, and
   registering 206 a time point for opening the measuring tank valve 124 as the filling start time point,
in case the level tank level LT-L is equal to or above the upper reference level UPP-REF,
   closing 208 the measuring tank valve 124 such that the sterilizing solution 102 from the measuring tank 106 is hindered from being transferred to the bath 108, and
   registering 210 a time point for closing the measuring tank valve 124 as the filling end time point,
determining 212 a filling start measuring tank level by retrieving the measuring level tank level LT-L for the filling start time point,
determining 214 a filling end measuring tank level by retrieving the measuring tank level data 112 for the filling end time point,
determining 216 the volume of sterilizing solution used between the filling start time point and the filling end time point by using the measuring tank level data 112 for the filling start time point and the filling end time point, and a cross-sectional area of the measuring tank 106, and
determining 218 the volume of sterilizing solution used per time unit by dividing the volume of sterilizing solution determined with a number of time units between the filling start time point and the filing end time point.

Optionally, in case the volume of sterilizing solution per time unit is below the lower risk threshold LOW-RISK,
transmitting 220 the sterility risk notification to the operator device 130.

Optionally, in case the volume of sterilizing solution per time unit is above the upper risk threshold UPP-RISK,
transmitting 222 the sterilizing solution residues risk notification to the operator device 130.

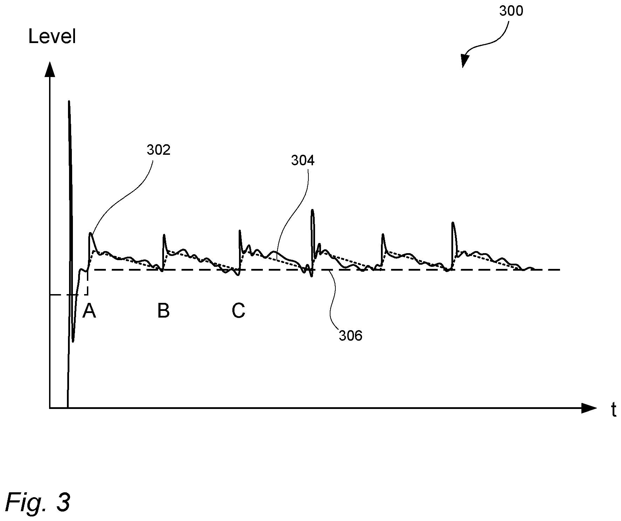

Fig. 3 illustrates a diagram 300 illustrating level measurements, reflected in the level tank level data 122, made by the level tank level detection device 120. As illustrated, an actual level signal 302 received via the level detection device 120 may contain noise. In addition, during start-up, there may be a start-up time during which no reliable level measurement can be made. Once being stabilized, the sterilizing solution fed into the bath via the measuring tank 106 is reflected in the actual level signal 302 as well as a filtered level signal 304, being a processed version of the actual level signal 302. As illustrated, releasing the sterilizing solution into the bath will result in that there is a steep increase in the filtered level signal 304. As the sterilizing solution is consumed, that is, absorbed by the web fed into the bath, the level in the level tank is decreasing as illustrated in the filtered level signal 304. As illustrated, a base line 306 may be provided. This base line may represent a level in the level tank 114 at which a fill-up of the sterilizing solution is to be made.

As illustrated, the level in the level tank 114 is following a cyclical pattern. In a first time point A, the sterilizing solution is starting to be filled into the bath from the measuring for a first time. After the sterilizing solution filled into the bath is consumed, in a second time point B, the sterilizing solution is filled from the measuring tank into the bath for a second time. After also this has been consumed, in a third time point C, the sterilizing solution is filled into the bath for a third time, and so on.

Since a cross-sectional area of the level tank is known, at least fixed and can be measured, the level in the level tank may be translated into a volume being held in the level tank at different time points. By taking into that a time difference between two time points is also known, or at least can be derived from control data being sent to the measuring tank valve 124, the consumption can be calculated for each cycle.

The volume being released from the measuring tank into the bath may be 20-40 ml, more preferably 30 ml. A level increase in the level tank 114 during a cycle may be 1 mm. The time difference between two time points, that is, a length of the cycle, may be 3 to 6 minutes, more preferably 4 to 5 minutes.

Even though the level detection device 110 may be a floating device. It is an alternative to use an ultrasound sensor for measuring the level of the level tank.

In addition to the above methods and apparatuses for measuring the volume of sterilizing solution used per time unit, there may also be provided an arrangement for providing of volume of sterilizing solution as described below:
Item 1. An arrangement for determining a consumption of a sterilizing solution in a sterilizing solution bath of a roll-based packaging machine, said arrangement comprising
   a measuring tank fluidly connected to the sterilizing solution bath,
   a measuring tank valve placed on a lower measuring tank pipe connecting the measuring tank to the bath, wherein in an open state the sterilizing solution in the measuring tank is allowed to pass into the bath, and in a closed state the sterilizing solution in the measuring tank is hindered to pass,
   a level tank fluidly connected to the sterilizing solution bath in at least two points such that the level tank and the sterilizing solution bath constitute communicating vessels,
   a level tank level detection device arranged to generate level tank level data representing a level tank level, and
   a control unit configured
   to transmit first control data to the measuring tank valve such that this is opened at a first time point, and
   to transmit second control data to the measuring tank valve such that this is closed at a second time point,
   to obtain first level tank level data pertaining to the first time point,
   to obtain second level tank level data pertaining to the second time point, and
   to determine the consumption based on the first and second level tank level data and the first and second time point.
Item 2. The arrangement according to item 1, wherein a cross-sectional area of the measuring tank is one tenth or less of a cross-sectional area of the bath.
Item 3. The arrangement according to item 1 or 2, wherein a time difference between the first and second time point is 2 to 6 minutes, more preferably 4 to 5 minutes.
Item 4. The arrangement according to any one of the preceding items, wherein the volume is 20 to 40 ml, more preferably 30 ml.
Item 5. The arrangement according to any one of the preceding items, wherein the arrangement comprises a roller placed in the bath for guiding a web in the bath, and wherein the cross-sectional area of the bath is a horizontal cross-sectional area above a lowermost part of the roller.
Item 6. A method for providing a volume of sterilizing solution into a sterilizing solution bath of a roll-based packaging machine, said arrangement comprising a measuring tank fluidly connected to the sterilizing solution bath, a measuring tank valve placed on a lower measuring tank pipe connecting the measuring tank to the bath, wherein in an open state the sterilizing solution in the measuring tank is allowed to pass into the bath, and in a closed state the sterilizing solution in the measuring tank is hindered to pass, and a control unit, said method comprising
   transmitting first control data to the measuring tank valve such that this is opened at a first time point, and
   transmitting second control data to the measuring tank valve such that this is closed at a second time point,
   obtaining first level tank level data pertaining to the first time point,
   obtaining second level tank level data pertaining to the second time point, and
   determining the consumption based on the first and second level tank level data and the first and second time point.
Item 7. The method according to item 6, wherein a cross-sectional area of the measuring tank is one tenth or less of a cross-sectional area of the bath.
Item 8. The method according to item 6 or 7, wherein a time difference between the first and second time point is 2 to 6 minutes, more preferably 4 to 5 minutes.
Item 9. The method according to any one of the preceding items, wherein the volume is 20 to 40 ml, more preferably 30 ml.
Item 10. The method according to any one of the preceding items, wherein the arrangement comprises a roller placed in the bath for guiding a web in the bath, and wherein the cross-sectional area of the bath is a horizontal cross-sectional area above a lowermost part of the roller.

From the description above follows that, although various embodiments of the invention have been described and shown, the invention is not restricted thereto, but may also be embodied in other ways within the scope of the subject-matter defined in the following claims.

## Claims

1. An arrangement (100) for measuring a volume (VOL) of sterilizing solution (102) used per time unit in a roll-based packaging machine (104), said arrangement (100) comprising
a measuring tank (106) fluidly connected to a sterilizing solution bath (108),
a measuring tank level detection device (110) arranged to generate measuring tank level data (112) representing a measuring tank level (MT-L),
a level tank (114) fluidly connected to the sterilizing solution bath (108) in at least two points (116, 118) such that the level tank (114) and the sterilizing solution bath (108) constitute communicating vessels,
a level tank level detection device (120) arranged to generate level tank level data (122) representing a level tank level (LT-L),
a measuring tank valve (124) placed on a lower measuring tank pipe (126) connecting the measuring tank (106) to the bath (108), wherein in an open state (OS) the sterilizing solution in the measuring tank (106) is allowed to pass into the bath (108), and in a closed state (CS) the sterilizing solution in the measuring tank (106) is hindered to pass, and
a control unit (128) configured
to retrieve the level tank level data (122),
in case the level tank level (LT-L) is equal to or below a lower reference level (LOW-REF),
to open the measuring tank valve (124) such that at least part of the sterilizing solution (102) in the measuring tank (106) is transferred to the bath (108), and
to register a time point for opening the measuring tank valve (124) as a filling start time point,
in case the level tank level (LT-L) is equal to or above an upper reference level (UPP-REF),
to close the measuring tank valve (124) such that the sterilizing solution (102) from the measuring tank (106) is hindered from being transferred to the bath (108), and
to register a time point for closing the measuring tank valve (124) as a filling end time point,
to determine a filling start measuring tank level by retrieving the measuring the level tank level (LT-L) for the filling start time point,
to determine a filling end measuring tank level (MT-L) by retrieving the measuring tank level data (112) for the filling end time point,
to determine the volume of sterilizing solution used between the filling start time point and the filling end time point by using the measuring tank level data (112) for the filling start time point and the filling end time point, and a cross-sectional area of the measuring tank (106), and
to determine the volume of sterilizing solution used per time unit by dividing the volume of sterilizing solution determined with a number of time units between the filling start time point and the filing end time point.

2. The arrangement (100) according to claim 1, wherein the measuring tank (106) is placed above the sterilizing solution bath (108) such that with the measuring tank valve (124) in the open state (OS) the sterilizing solution in the measuring tank (106) is released down into the sterilizing solution bath (108) via the valve (124), and wherein the measuring tank valve (124) is configured such that in case there is no electrical power provided, the valve (124) is in the open state (OS).

3. The arrangement (100) according to any one of the preceding claims,
wherein the measuring tank level detection device (110) and/or the level tank level detection device (120) is a floating device.

4. The arrangement (100) according to any one of the preceding claims,
wherein the sterilizing solution is hydrogen peroxide.

5. The arrangement (100) according to any one of the preceding claims,
wherein a total volume of the bath (108) is 5-20 times a total volume of the level tank (114).

6. The arrangement (100) according to any one of the preceding claims,
wherein a total volume of the measuring tank (106) is greater than the total volume of the level tank (114).

7. The arrangement (100) according to any one of the preceding claims,
wherein the volume of sterilizing solution per time unit is 0.3 to 2 liters per hour.

8. The arrangement (100) according to any one of the preceding claims, wherein the control unit (128) is further configured to
in case the volume of sterilizing solution per time unit is below a lower risk threshold (LOW-RISK),
to transmit a sterility risk notification to an operator device (130).

9. The arrangement (100) according to any one of the preceding claims, wherein the control unit (128) is further configured to
in case the volume of sterilizing solution per time unit is above an upper risk threshold (UPP-RISK),
to transmit a sterilizing solution residues risk notification to the operator device (130).

10. A roll-fed packaging machine (104) comprising the arrangement according to any one of the preceding claims.

11. A method (200) for measuring a volume (VOL) of sterilizing solution (102) used per time unit in a roll-based packaging machine (104) by using an arrangement (100), said arrangement comprising a measuring tank (106) fluidly connected to a sterilizing solution bath (108), a measuring tank level detection device (110) arranged to generate measuring tank level data (112) representing a measuring tank level (MT-L), a level tank (114) fluidly connected to the sterilizing solution bath (108) in at least two points (116, 118) such that the level tank (114) and the sterilizing solution bath (108) constitute communicating vessels, a level tank level detection device (120) arranged to generate level tank level data (122) representing a level tank level (LT-L), a measuring tank valve (124) placed on a lower measuring tank pipe (126) connecting the measuring tank (106) to the bath (108), wherein in an open state (OS) the sterilizing solution in the measuring tank (106) is allowed to pass into the bath (108), and in a closed state (CS) the sterilizing solution in the measuring tank (106) is hindered to pass, and a control unit (128), said method (200) comprising
retrieving (202) the level tank level data (122),
in case the level tank level (LT-L) is equal to or below a lower reference level (LOW-REF),
opening (204) the measuring tank valve (124) such that at least part of the sterilizing solution (102) in the measuring tank (106) is transferred to the bath (108), and
registering (206) a time point for opening the measuring tank valve (124) as a filling start time point,
in case the level tank level (LT-L) is equal to or above an upper reference level (UPP-REF),
closing (208) the measuring tank valve (124) such that the sterilizing solution (102) from the measuring tank (106) is hindered from being transferred to the bath (108), and
registering (210) a time point for closing the measuring tank valve (124) as a filling end time point,
determining (212) a filling start measuring tank level by retrieving the measuring level tank level (LT-L) for the filling start time point,
determining (214) a filling end measuring tank level by retrieving the measuring tank level data (112) for the filling end time point,
determining (216) the volume of sterilizing solution used between the filling start time point and the filling end time point by using the measuring tank level data (112) for the filling start time point and the filling end time point, and a cross-sectional area of the measuring tank (106), and
determining (218) the volume of sterilizing solution used per time unit by dividing the volume of sterilizing solution determined with a number of time units between the filling start time point and the filing end time point.

12. The method (200) according to claim 11, wherein a total volume of the bath (106) is 5-20 times a total volume of the level tank (114).

13. The method (200) according to any one of the claims 11 to 12, further comprising
in case the volume of sterilizing solution per time unit is below a lower risk threshold (LOW-RISK),
transmitting (220) a sterility risk notification to an operator device (130).

14. The method (200) according to any one of the claims 11 to 13, further comprising
in case the volume of sterilizing solution per time unit is above an upper risk threshold (UPP-RISK),
transmitting (222) a sterilizing solution residues risk notification to the operator device (130).

15. A computer program product comprising instructions which, when the program is executed by a computing device, causes the computer to carry out the method (200) according to any one of the claims 10 to 14.
